(19) **Europäisches Patentamt / European Patent Office / Office européen des brevets**

(11) **EP 1 856 270 B1**

(12) **EUROPEAN PATENT SPECIFICATION**

(45) Date of publication and mention of the grant of the patent:
**25.12.2013 Bulletin 2013/52**

(51) Int Cl.:
*C12P 19/18* (2006.01)     *A23L 1/09* (2006.01)
*C13K 13/00* (2006.01)

(21) Application number: **06720745.6**

(22) Date of filing: **15.02.2006**

(86) International application number:
**PCT/US2006/005210**

(87) International publication number:
**WO 2006/088884 (24.08.2006 Gazette 2006/34)**

(54) **Methods of making syrups**

Verfahren zur Herstellung von Sirupen

Procédés de fabrication de sirops

(84) Designated Contracting States:
**AT BE BG CH CY CZ DE DK EE ES FI FR GB GR HU IE IS IT LI LT LU LV MC NL PL PT RO SE SI SK TR**

(30) Priority: **15.02.2005 US 653008 P**

(43) Date of publication of application:
**21.11.2007 Bulletin 2007/47**

(73) Proprietor: **CARGILL, INCORPORATED**
**Wayzata, Minnesota 55391 (US)**

(72) Inventors:
• **CARLSON, Ting, L.**
  **Dayton, Minnesota 45458 (US)**
• **WOO, Anton**
  **Huber Heights, Ohio 45424 (US)**
• **ZHENG, Guo-hua**
  **Centerville, Ohio 45459 (US)**

(74) Representative: **Wibbelmann, Jobst**
**Wuesthoff & Wuesthoff**
**Patent- und Rechtsanwälte**
**Schweigerstrasse 2**
**81541 München (DE)**

(56) References cited:
**WO-A1-2004/023894      WO-A1-2004/068966**
**DE-B3- 10 209 629**

• SANZ ET AL: "Prebiotic properties of alternansucrase maltose-acceptor oligosaccharides", JOURNAL OF AGRICULTURAL AND FOOD CHEMISTRY, vol. 53, 22 June 2005 (2005-06-22), pages 5911-5916, XP002647292,
• COTE G.L. ET AL.: 'Acceptor Reactions of Alternansucrase from Leuconostoc mesenteroides NRRL B-1355' CARBOHYDRATE RESEARCH vol. 111, no. 1, 16 December 1982, pages 127 - 142, XP003000268
• LÓPEZ-MUNGUÍA ET AL: "Production and purification of alternansucrase, a glucosyltransferase from Leuconostoc mesenteroides NRRL B-1355, for the synthesis of oligoalternans", ENZYME AND MICROBIAL TECHNOLOGY, vol. 15, 1993, pages 77-85, XP008018878,

**Description**

**Background**

Corn syrups for use in the production of beverages (e.g., sports drinks) and other food applications are known. It would be desirable, however, to have available for use in beverages and other food applications, as required, a product having sweetness and functionality that is similar to corn syrup, yet having a lower glycemic index.

**Summary**

Provided herein are efficient methods of making substantially clear low-glycemic syrups (LGS) that comprise alternan oligosaccharides. These syrups have a relatively low glycemic index and are additionally useful in applications where increased clarity are desired. These qualities are particularly beneficial in food and beverage formulations.

The methods of making the substantially clear LGS as defined in the claims involve reacting more than one substrate (initial sucrose and one or more initial acceptors selected from a sugar or sugar alcohol having free hydroxyl groups at one or more of carbon positions numbers 2, 3 and 6 that can accept a glucose unit from sucrose) at a concentration of at least 40 % w/w with at least one alternan sucrase enzyme at temperatures of greater than 45°C. In additional embodiments the substrate is at a concentration of at least about 41, 42, 43, 44, 45, 46, 47, 48, 49, 50, 51, 52, 53, 54, or 55% w/w. In additional embodiments that substrate and enzyme can be reacted at temperatures of greater than 46, 47, 48, 49, 50, or 51°C.

In some embodiments the methods involve reacting one or more of the alternan sucrases with the substrate for relatively short period of time, such as less than 12 hours, or less than 11, 10, 9, 8, or 7 hours.

Substantially clear LGS made at temperatures greater than 45°C and substrate concentrations of at least 40 %w/w are typically visually clearer when compared to syrups made at lower temperatures and lower substrate concentration. Clarity can also be determined using the test procedure described herein. In some embodiments the substantially clear LGS when adjusted to a concentration of 20 % w/w will display a transmittance at 650 nm of greater than 93%, or greater than 94, 95, 96, 97, 98, or 99%.

In some embodiments the substantially clear LGS comprises less than 0.5% alternan that is greater than DP 12 (degree of polymerization of glucose units).

In some embodiments the methods provide substantially clear LGS that have low viscosity, such as a viscosity of less than 14,000 cps at 80°F and 77% dry solid concentration. In other embodiments the substantially clear LGS when measured at 80°F and 77% dry solid concentration will display a viscosity of less than 10000, 9000, 8000, 7000, 6000, 5000 or 4,000 cps.

In some embodiments the alternan sucrase enzyme used can be made from recombinant methods such as those described in US6,570,065. In additional embodiments the recombinant enzyme will be smaller or truncated when compared to the full-length enzyme "Characterization of alternansucrase from Leuconostoc mesenteroides NNRL B-1355: rational and random approach to design of novel glucansucrase" Gilles Joucla, Doctroal Dissertation, Ingenier INSA, Toulouse, France, 2003.

In another aspect, the invention provides a method of making a food or beverage comprising mixing one or more ingredients with a substantially clear syrup made by the method of making a substantially clear syrup as defined in the claims.

WO2004023894 (A1) discloses a process for preparing a low glycemic index food or beverage composition comprising incorporating into the food or beverage composition a low-glycemic sweetener comprising a blend of sucrose and a component selected from the group consisting of a syrup and syrup solids comprising an acceptor selected from the group consisting of a sugar and a sugar alcohol having free hydroxyl groups at one or more of carbon positions numbers 2,3 and 6 that can accept a glucose unit from sucrose, said blend having been reacted with a glucansucrase enzyme.

WO2004068966 (A1) discloses a process for elongating chain length of isomalto-oligosaccharides characterised in that said process comprises an enzymatic transfer reaction between sucrose and isomalto-oligosaccharides present in a carbohydrate syrup. In particular, said process comprises the following steps: a) Using one or more isomalto-oligosaccharide producing enzymes to convert a malto-oligosaccharide syrup into a carbohydrate syrup containing isomalto-oligosaccharides, b) Adding sucrose to the carbohydrate syrup, c) Adding glucansucrase, d) Producing a syrupy mixture by converting the isomalto-oligosaccharides in the carbohydrate syrup into isomalto-oligosaccharides with elongated chain length.

Sanz et al. (J. Agric. Food Chem. 2005, 53, 5911-5916) disclose a process of providing oligosaccharides from the reaction between sucrose and maltose by catalysis with an alternansucrase isolated from *Leuconostoc mesenteroides* NRRL B-21297.

These and other objects and advantages of the present invention will be apparent to those skilled in the art from the following detailed description and claims.

## Detailed Description

### I. Methods of making syrups

The substantially clear LGS described herein display a variety of physical characteristics. More specifically, alternan oligosaccharides can be produced from an acceptor and sucrose that are reacted with one or more alternan sucrase enzymes that will transfer glucose units from sucrose to an acceptor carbohydrate and will release fructose and glucose oligosaccharides of various lengths. The resultant product may have a level of sweetness similar to that of a corn syrup, and a mouth-feel and functionality similar to that of corn syrup. In addition, and more significantly for the present methods, the resulting product may be characterized in some embodiments by having a lower glycemic index as compared to the combination of the substrates (sucrose and acceptors) that are not reacted with enzyme. These syrups are referred to as substantially clear low-glycemic syrups (LGS).

The acceptor is selected from the group consisting of a sugar or a sugar alcohol having free hydroxyl groups at one or more carbon positions numbers 2, 3 and 6 that can accept a glucose unit from sucrose. The acceptor can be in the form of syrup or syrup solids. Exemplary of the syrups or syrup solids suitable for use herein are maltose, maltotriose, panose, high maltose (over 40%) corn syrup, medium to low DE (dextrose equivalent) corn syrup, raffinose, cellobiose, maltitol, maltotriose, maltotetrose, glucose, isomaltose, isomaltitol, barley syrup and syrup solids, rice syrup and syrup solids, lactose, whey permeate, tapioca starch syrup and syrup solids, nigerose, kojibiose, isomaltooligosaccharide, hydrogenated starch syrup, potato starch syrup and syrup solids, corn syrup and syrup solids, and the like. Exemplary of the syrups that are suitable for use in the blends are, but not limited to, SATINSWEET™, available from Cargill, Incorporated, that contains minimal 55 to 70 weight % maltose and 45 to 30% weight % of glucose and other glucose-containing oligomers. In one embodiment, the syrup or syrup solids used comprise an amount of from about 2 to about 99% by weight of maltose.

The alternansucrase enzymes that can be used in the reaction to produce the LGS include, but not limited to, *Leuconostoc Mesenteroides* (LM) strains NRRL B 1355,23185, 23186, 23188, 23311, and 21297, and other enzymes provided herein. These enzymes can be additionally cloned and expressed recombinantly, such as described in Gilles Joucla, Doctroal Dissertation, Ingenier INSA, Toulouse, France, 2003. These strains can be cultured and the enzymes can be isolated using any method known in the art, such as the methods provided below.

The physical characteristics of the syrups produced can be altered by using different reaction conditions. For example, a substantially clear LGS can be produced by reacting one or more of the alternan sucrase enzymes in a reaction with relatively high concentration of sucrose and acceptor. Moreover, the efficiency of the reaction can be increased by reacting at a temperature of greater that 45°C. This allows for less enzyme to be needed in order to achieve completion (when the remaining substrate is <2% of the total carbohydrates w/w basis) of the reaction, and the reaction can be completed in a shorter period of time. For example, the reaction, can be carried out at a temperature of greater than 45° C for a period of less than 4, 5, 6, 7, 8, 9, 10, 11, 12, 13, 14, 15, 16, 17, or less than 20 hours. The increased temperature may also lessen the risk of microbial contamination in some embodiments.

The characteristics of LGS can be altered by controlling the ratio of sucrose to acceptor. Generally, the glycemic index of the substantially clear LGS will decrease as the ratio of sucrose to acceptor increases up to a ratio of about 12:1. For example, it is expected that a product made using a ratio of 1:1 (sucrose to acceptor) will have a higher glycemic index than that of a product made using a ratio of 4:1 (sucrose to acceptor). In some embodiments a ratio of from about 8:1 1 to about 11:1 are used. In other embodiments, the methods comprise making LGS using ratios of sucrose to acceptor of at least 4:1, 5:1, 6:1, 7:1, 8:1, 9:1, and 10:1. Accordingly, the invention also provides food and beverage products made by such methods.

The LGS can also be characterized by the linkages between the glucose molecules in the glucose oligosaccharide. In some embodiments the glucose oligosaccharide has both alpha 1,3 and alpha 1,6 linkages. In some embodiments, the glucose oligosaccharide also contains other linkages such as alpha 1,4. In some embodiments that LGS will have at least 20% alpha 1,3 linkages. In other embodiments the LGS will have at least 20% alpha 1,3 linkages and at least 20% alpha 1,6 linkages.

In some embodiments, the substantially clear LGS is subsequently processed to remove a portion of, or all of, the fructose, thus yielding a LGS that is fructose depleted. Fructose can be removed from the LGS using any method known in the art, for example by using column chromatography. Generally, the LGS contains less than 50% fructose. The substantially clear LGS and/or the fructose depleted LGS can further be hydrogenated to make a non-reducing syrup.

In other embodiments an oligoalteran with a degree of polymerization of 7 to 12 is provided that is a slowly but fully digestible carbohydrate which makes LGS a unique sweetener that offers a low glycemic response and sustained energy without any intestinal discomfort or side effects.

**II Products and Blends including a substantially clear low-glycemic syrup (LGS)**

The substantially clear LGS described herein can be blended with one or more of a variety of other ingredients and can be sold to formulators as blends, or the components for the blends can be provided to the formulator separately and the formulator can blend them while making a final food product. Substantially clear LGS can be blended with one or more other ingredients such as vitamins, minerals, sugar alcohols, high intensity sweeteners, flavors, flavor enhancers, and other conventional sweeteners to provide the desired nutritional impact as well as the desired flavor. The creation of blends with substantially clear LGS is expected to improve the homogeneity of the end products.

Vitamins that can be blended with substantially clear LGS include any of a group of organic substances other than proteins, carbohydrates, fats, minerals, and organic salts wllich are essential for normal metabolism, growth, and development of the body. Vitamins include compounds such as A, D, E, I, biotin, choline, folic acid, and nicotinic acid.

Mineral compounds that can be blended with substantially clear LGS include inorganic compounds of mineral elements, which constitute the mineral constituents of the body. Mineral salts and water are excreted daily from the body and, therefore, need to be replenished. These must be replaced through food or supplement intake. Examples of minerals include Ca, Fe, P. Na, Cu, K, and Mg.

Flavors and/or flavor enhancers can be also blended with LGS. For example dihydroxybenzoic acid (DHB, including all isomers) as well as flavors such as peppermint, cocoa, and vanilla.

Sugar alcohols can be blended with LGS and used to impart sweetness to a particular food product and in many instances the sugar alcohol will not contribute as greatly to the caloric content of the product when compared to conventional sweeteners. Sugar alcohols are characterized by the presence of a hydroxyl group on a ketose sugar or hexose sugar. Examples of sugar alcohols that can be blended with the LGS sweeteners  described herein include sorbitol, lo manitol, xylitol, lactitol, maltitol, isomalt, hydrogenated starch hydrolysate, and erythritol.

LGS disclosed herein can also be blended with high-intensity sweeteners. High-intensity sweeteners are agents that exhibit sweetening powers at very low concentrations. Examples of high-intensity sweeteners that can be blended with the LGS compositions described herein include saccharin, cyclamate, aspartame, monatin, alitame, acesulfame potassium, sucralose, thaumatin, stevioside, and glycynhizin.

Additional examples of uses and products that can be made using substantially clear LGS are included in WO2004023894A1 and WO2005089483A2.

The following examples provide specific embodiments and one of ordinary skill in the art will recognize that these examples are given for the purpose of illustration only not to be construed as limiting the scope of what is claimed any way.

**Examples**

Note that NRRL B 30821 and NRRL B 30894 have not been made available, and are therefore not part of the claimed scope.

**Example 1, Method of making enzyme solution**

Enzyme preparations were generated by growing *Leuconostoc Mesenteroides* NRRL B 30821 or NRRL B 30894, on media containing 10 g/L yeast extract, 5 g/L potassium phosphate, 0.2 g/L magnesium sulfate (heptahydrate), 0.1 g/L manganese sulfate (monohydrate), 0.02 g/L calcium chloride, 0.01 g/L sodium chloride, 0.01 g/L ferrous sulfate (heptahydrate) and 20-40 g/L glucose from various sources. *Leuconostoc Mesenteroides* NRRL B 21297 culture was grown in the same medium except with 2% sucrose and 2% high maltose corn syrup (65% maltose) as the carbon source. Cultures were grown at 27-30°C and with pH control at 6.0 (with 10% NaOH) until carbohydrate was consumed. Once the carbon source was depleted, cells were removed by centrifugation at 12,200 x g at 4°C for 30 minutes. The clarified culture supernatant was concentrated by ultrafiltration through a 50,000 molecular weight cutoff (50 kD MWCO) membrane to obtain a 10-fold enzyme concentrate by volume. Both strains NRRL B 30821 and NRRL B 30894 were derived from *Leuconostoc Mesenteroides* NRRL B 21297 by chemical mutagenesis and express alternan sucrase constitutively when grown on glucose medium.

**Example 2, Assay to determine digestibility**

The digestibility of the resulting syrup was determined by an in vitro digestion assay. 2 mL of 8% testing syrup was mixed with 10 $\mu$L of glucoamylase (Optidex L-400 from Genencor International, Palo Alto, CA), and 2 mL of 0.019 M acetate buffer, pH=4.5, and incubated at 60°C for 16-20 hours. After the digestion, 2 mL of the syrup was withdrawn and mixed with 2 mL of 0.05 M phosphate buffer, pH=6.5, 0.1 mL of 1% NaN3, and 0.1 g of rat intestinal powder (Catalog# I-1630, Sigma-Aldrich Fine Chemicals, St. Louis, MO, USA) and incubated at 37°C for up to 24 hours. At each time point, 0.5 mL of the mixture was mixed with 1 mL of 1.2 N HCl to stop further reaction. The amount of glucose release by glucoamylase and rat intestinal enzyme hydrolysis was determined by HPLC using an Aminex HPX-87H column, available from Bio-Rad Laboratories (Hercules, CA), with 0.01N (normal) sulfuric acid as the mobile phase at 0.6 ml/min and 60°C. Digestibility was expressed as % glucose released from the total gluco-oligosaccharides and is calculated by the following equation:

$$\% \text{ Theoretical glucose release} = [\text{glucose}]/[\text{fructose}]*0.45/0.55*100$$

Alternatively, the initial glucoamylase treatment step can be omitted.

**Example 3, Enzyme assay and unit definition**

To 2.5 mL of 40% (w/v) sucrose to maltose at a 9:1 ratio and 0.1 ml of 1.5 M citrate buffer, pH 5.5, 0.1 to 2.4 mL of concentrated enzyme was added. The volume was brought up to 5.0 ml with nanopure water and the reactions were incubated at 37°C. After 1 hour, 0.5 mL of each reaction was placed in a 90-100°C water bath for 5 minutes. After a short cooling period, 1.0 mL of nanopure water was added to the 0.5 mL samples and 0.05-0.1 g of an ion exchange mixture consisting of Dowex 66 and Dowex 88 (Dow Chemical Co., Midland, MI) was added. The samples were inverted for 2-3 minutes and were filtered through 0.45 $\mu$m nylon filter (Whatman, Clifton, NJ) into HPLC vials. Samples were run on an HPLC through an HPX-87C carbohydrate column (300mm X 7.8 mm) (Bio-Rad, Hercules, CA) at 85°C with water as the mobile phase at 0.9 mL/min. Calculations were based upon the percent area of fructose relative to the area of total sugars. The calculation is as follows: Percent fructose * 1 g total sugar in 5 mL reaction/ 1 h/ volume of enzyme broth used = g fructose/h/mL of activity.

Values are then converted to $\mu$mole fructose/min/ml using the formula weight of fructose and metric conversions. One unit is defined as the release of 1 $\mu$mol of fructose/minute at 37°C and values are reported in U/mL.

*Example 4: Low glycemic syrup sugar profile analysis*

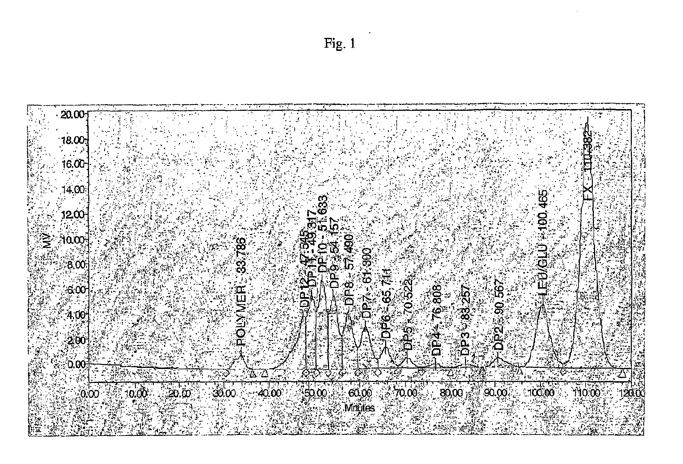

The oligosaccharide profile of low glycemic syrup is analyzed by a HPLC method as described below. A sample of low glycemic syrup is diluted with deionized water to 5-10% dry solids, de-ashed with ion exchange resins (Dowex 66/Dowex 88, Dow Chemical Co., Midland, MI), and filtered through a 0.45 micron filter before injection into the HPLC for sugar analysis. The oligosaccharide separation is accomplished by using two BioRad Aminex HPX-42A, 300-7.8 mm, columns (Hercules, CA) in series at 65°C and with a refractive index detector. Water is used as the eluent at a flow rate of 0.2 ml/min. The chromatogram of a typical low glycemic syrup sample (PDF8) is shown in Fig. 1. The sequential peaks shown on the chromatogram are assigned as oligomers of increasing degree of polymerization (DP). For example, DP3 is a trisaccharide and DP4 is a tetrasaccharide.

The peak that elutes in the shortest time at the far left is assigned as a polymer peak with a molecular weight of at least 250,000.

**Example 5, Enzymatic conversion of sucrose/maltose substrate (sucrose:maltose ratio = 9:1) to specialty clear syrup at high temperatures**

Substantially clear LGS with slow digestibility and low viscosity can be made at temperatures higher than 45° C. At the elevated temperatures, the rate of converting sugar substrates to the clear syrup is greatly increased. Because of increased reaction rates at high temperatures, the enzyme usage, conversion time length and the risk of microbial contamination during processing are greatly reduced. This is of particular importance in commercial production, offering economic advantages for production of the substantially clear LGS.

An enzyme preparation was obtained from *Leuconostoc Mesenteroides* NRRL B 30821 as described in Example 1. The enzyme preparation had an activity of 92.5 Units/mL as defined in Example 3. The specific gravity of the enzyme preparation and the water was assumed at 1 g/mL. Crystalline sucrose and maltose, both purchased from Sigma-Aldrich Fine Chemicals (St. Louis, MO, USA), were dry mixed at sucrose:maltose ratio of 9:1 on a dry weight basis and ground in a coffee grinder to obtain a homogenous powdery substrate mixture, referred to as the substrate hereafter. The sugar substrate was dissolved in de-ionized water to final concentrations of 20, 30, 40 and 50% weight-by-weight including the addition of the enzyme preparation in 50-mL capped polypropylene test tubes. An aliquot of 0.17 mL of the enzyme preparation was added to each test tube, to yield varying enzyme dosages (Table 1). The total weight of the substrate, water and the enzyme was 8.50 g in each test tube. The test tubes were placed in water baths set at 37 ° C, 45 ° C, 50 ° C and 55 ° C for 30 min for the substrate solution to be equilibrated to respective temperatures before enzyme addition.

Table 1. Substrate concentration, enzyme dosage and the amounts of substrate and water contained in each test tube. 0.17 mL of enzyme was added to each test tube (equivalent to 15.7 total units).

| Substrate concentration | Substrate | Water | Enzyme dosage |
|---|---|---|---|
| % w/w | g/tube | mL/tube | Unit/g substrate |
| 20 | 1.70 | 6.63 | 9.25 |
| 30 | 2.55 | 5.78 | 6.17 |
| 40 | 3.40 | 5.93 | 4.63 |
| 50 | 4.25 | 4.08 | 3.70 |

After the addition of enzyme preparation, the test tubes were placed back into their respective water baths at 37 ° C, 45 ° C, 50° C and 55 ° C. After 20 hours of incubation, a small sample was drawn from each test tube. The remaining substrate in the samples was analyzed by HPLC as described in example 3 except the percent area of substrate was calculated against the total sugar areas. The average reaction rate during the 20 hr reaction was calculated as $\mu$mols of substrate depleted per hour per unit of enzyme ($\mu$mols substrate/hr/unit).

Table 2, below, provides the results of the reaction and shows that reactions at higher temperature and higher substrate concentration showed higher substrate conversion and higher reaction rate. As a result, one of ordinary skill in the art will appreciate that less enzyme is needed to complete the reaction in less time at high temperatures and high substrate concentrations than at lower substrate concentrations and lower temperatures. For example, 97% substrate utilization was achieved at 50% substrate concentration and 50 ° C with 3.7 U/g enzyme, 98% substrate completion at 40% concentration and 45 ° C with 4.63 U/g enzyme as compared to 98% substrate depletion at 30% concentration, 37 ° C with 6.17 U/g enzyme within 20 hours. It required 9.25 U/g enzyme to complete a 20% concentration reaction at 37 °C. From an industrial operation standpoint, reactions at high substrate concentration also offer the benefits of reduced usage of water and energy.

From this experiment, one of ordinary skill in the art will also appreciate that a much longer time will be required to complete the reactions at high substrate concentration and low temperature (e.g. 37°C), due to a slow reaction rate.

Table 2. Effects of substrate concentration and temperature on substrate conversion and reaction rate

| substrate concentration | enzyme dosage | Reaction temperature | | | | | | | |
| --- | --- | --- | --- | --- | --- | --- | --- | --- | --- |
| | | 37°C | | 45°C | | 50°C | | 55°C | |
| | | substrate depletion | reaction rate | substrate depletion | reaction rate | substrate depletion | reaction rate | substrate depletion | reaction rate |
| % w/w | U/g | % | umol/hr/ unit | % | umol/hr/ unit | % | umol/hr/ unit | % | umol/hr/ unit |
| 20 | 9.25 | 99 | 15.6 | 55 | 8.7 | 22 | 3.5 | 17 | 2.7 |
| 30 | 6.17 | 98 | 23.2 | 78 | 18.5 | 37 | 8.8 | 20 | 4.7 |
| 40 | 4.63 | 89 | 28.0 | 98 | 30.9 | 64 | 20.2 | 32 | 10.1 |
| 50 | 3.70 | 74 | 29.2 | 94 | 37.1 | 97 | 38.3 | 50 | 19.8 |

**Example 5, Chemical and physiological properties of clear syrups made at high temperatures**

An enzyme preparation was obtained from *Leuconostoc Mesenteroides* NRRL B 30821 as described in Example 1. The enzyme preparation had activity of 92.5 Units/mL as defined in Example 3. The specific gravity of the enzyme preparation and the water was assumed at 1g/mL. Crystalline sucrose and maltose, both purchased from Sigma-Aldrich Fine Chemicals (St. Louis, MO, USA), were dry mixed at sucrose: maltose ratio of 9:1 on dry weight basis and ground in a coffee grinder to obtain a homogeneous powdery substrate mixture, refereed as the substrate hereafter. The sugar substrate was dissolved in de-ionized water to final concentrations of 50 and 60% weight-by-weight including the addition of the enzyme preparation in 50-mL capped polypropylene test tubes. Aliquots of the enzyme preparation were added to each test tube to obtain final enzyme dosages of 2U/g, 5U/g and 8U/g. The total weight of the substrate, water and the enzyme was 20 g in each test tube. The test tubes were placed in water baths set at 50 °C for 30 min for the substrate solution to be equilibrated to respective temperatures before enzyme addition. After 48 hours at 50 ° C, the samples were analyzed for their sugar composition using dual  Aminex HPX-42A HPLC column described in Example 3. Digestibility of the samples were determined my method described in Example 2.

In general, syrups made at high temperature and high substrate concentration contained less polymer and more leucrose, but similar amounts of gluco-oligosaccharides (Table 3). Because the *in vitro* digestion rate was largely attributable to the contents of oligosaccharides, syrups made at high temperature and high substrate concentration showed similar *in vitro* digestibility as that made at a low temperature and low substrate concentration. The *in vitro* digestion rate results showed similar digestion rates among the samples tested. Under the same digestion condition, maltose or malto-oligosaccharides is 100% digested within 8 hours (data not shown).

Table 3. Sugar profile and in vitro digestibility of clear syrup made at high temperatures in comparison with that made at a low temperature

| Reaction condition | | | Sugar profile | | | | | | % Digested after 8hours |
|---|---|---|---|---|---|---|---|---|---|
| Temperature | Concentration | Enzyme dosage | Fructose | Leucrose | DP2 | DP3-6 | DP7-12 | polymers | |
| °C | % w/w | U/g | % | % | % | % | % | % | % |
| 37 (control) | 20 | 9 | 40.5 | 7.3 | 1.3 | 5.5 | 44.1 | 1.4 | 34.6 |
| 50 | 50 | 5 | 38.4 | 9.7 | 2.2 | 8.9 | 40.8 | 0.0 | 37.5 |
| | 50 | 8 | 38.5 | 10.9 | 2.3 | 8.5 | 39.5 | 0.0 | 37.9 |
| | 60 | 2 | 38.6 | 12.0 | 2.5 | 9.3 | 37.4 | 0.1 | 39.1 |
| | 60 | 5 | 38.0 | 12.2 | 2.1 | 10.4 | 37.1 | 0.2 | 39.6 |
| | 60 | 8 | 37.2 | 13.3 | 3.0 | 11.6 | 34.9 | 0.0 | 43.4 |

**Example 6, Clarity of syrups made at high temperatures**

Because of reduced polymer concentrations, syrups made at high temperatures exhibited another important characteristic, in that they are clear.

Syrup samples produced at 20% (w/w) substrate concentration and 37 °C at the Cargill Process Development Facility in Savage (MN, USA), PDF03, PDF04, PDF06, PDF08, and PDF10, were compared to those produced at 50% (w/w) substrate concentration and 50°C. A sample produced from PDF07 by ultrafiltration (50kD MWCO) to remove alternan polymer was also included in the comparison. All samples were diluted with de-ionized water from ~80 % dry solid to 50, 40, 30, 20, 10 and 5 % dry solid and light transmittance measured with a spectrophotometer (Hewlett Packard, Model 8453) at 650 nm using a 1-cm cell. Prior to measuring the transmittance of the test samples the spectrophotometer was calibrated with de-ionized water. As shown in Table 4, all the samples made at 20% substrate concentration and 37°C except PDF07 with the polymer removed by ultrafiltration had light transmittance of less than 96.1% and looked opaque at concentrations of 40 % dry solid or below. Conversely, the sample made at high temperature was as clear as that with polymer removed by ultrafiltration throughout the entire concentration range (from 0.5 % dry solid to 85 % dry solid).

Table 4. Light transmittance of a syrup made at high temperature as compared with those made at low temperature with or without polymer removed by ultrafiltration.

| Reaction condition | 50°C 50% w/w | 37°C 20% w/w | | | | | |
|---|---|---|---|---|---|---|---|
| Solution % solid | | PDF07 polymer removed | PDF03 | PDF04 | PDF06 | PDF08 | PDF10 |
| 40 | 99.45 | 99.12 | 94.15 | 92.46 | 96.06 | 94.06 | 80.14 |
| 30 | 98.51 | 98.19 | 91.88 | 90.46 | 92.91 | 90.95 | 77.75 |
| 20 | 96.86 | 98.13 | 92.12 | 89.95 | 91.54 | 90.9 | 79.05 |
| 10 | 96.93 | 97.88 | 93.36 | 90.83 | 92.18 | 90.89 | 85.71 |
| 5 | 99.35 | 99.78 | 96.8 | 95.4 | 96.3 | 96.3 | 91.37 |

**Example 7, Syrup made with NRRL B 30894 at high temperature**

Enzyme preparation from strain *Leuconostoc Mesenteroides* NRRL B 30821 and strain NRRL B 30894 was done according to Example 1. Syrup batch-PDF12 was made with 3 U/g substrate of enzyme from *Leuconostoc Mesenteroides* NRRL B 30894 at 50°C and 50% total sugar at a sucrose to maltose ratio of 9. Syrup batches PDF6 and PDF7 were made with NRRL B 30821 enzyme at a dosage of 6 U/g substrate at 37°C and 20% total sugar with a sucrose to maltose ratio of 9. The digestion assay was done as described in Example 2 without the glucoamylase pretreatment step. The viscosity measurement was performed at various temperatures with two different lots of syrup at 77% dry solid using a Brookfield DV-E Viscometer (Brookfield Engineering Labs, Inc., Middleboro, MA).

Syrup batches PDF6 and PDF7 were opaque and syrup batch PDF12 was a clear syrup. The sugar profile of the two types of syrups made at low (PDF7) and high temperature (PDF12) is shown in Table 6 and showed there was no polymer present in syrup PDF12. The clear syrup had a significantly lower viscosity as compared to the opaque syrup at the same dry solid levels (Table 7) and is digested by rat intestinal powder at a similar rate as the opaque syrup (Table 8).

Table 6: Sugar profile of clear and opaque syrup (in % of total sugar)

| in % | Fructose | Leucrose | DP2 | DP3 | DP4 | DP5 | DP6 | DP7 |
|---|---|---|---|---|---|---|---|---|
| 1st sample | 38.99 | 13.06 | 2.66 | 1.08 | 1.07 | 1.41 | 2.31 | 4.78 |
| drum 1 | 38.27 | 13.04 | 2.8 | 1.25 | 1.23 | 1.53 | 2.42 | 4.82 |
| drum 2 | 38.31 | 13.08 | 2.81 | 1.27 | 1.24 | 1.53 | 2.4 | 4.83 |
| drum 3 | 38.38 | 13.05 | 2.81 | 1.25 | 1.25 | 1.54 | 2.4 | 4.82 |
| PDF12 | 38.53 | 13.08 | 2.74 | 1.19 | 1.17 | 1.47 | 2.36 | 4.8 |
| drum 4b | 38.36 | 13.07 | 2.81 | 1.26 | 1.23 | 1.52 | 2.38 | 4.81 |

(continued)

| in % | Fructose | Leucrose | DP2 | DP3 | DP4 | DP5 | DP6 | DP7 |
|---|---|---|---|---|---|---|---|---|
| drum 5 | 38.17 | 13.01 | 2.79 | 1.28 | 1.24 | 1.54 | 2.39 | 4.85 |
| drum 6 | 38.17 | 13.01 | 2:79 | 1.28 | 1.24 | 1.54 | 2.39 | 4.85 |
| bucket 1 | 38.27 | 13.02 | 2.79 | 1.27 | 1.25 | 1.55 | 2.41 | 4.85 |
| PDF7 | 42.22 | 7.46 | 1.61 | 0.75 | 0.88 | 1.87 | 3.25 | 5.95 |
| in % | DP8 | DP9 | DP10 | DP11 | DP12 | Polymer | | |
| PDF12 | 5.95 | 7.53 | 8.28 | 6.48 | 6.5 | 0 | | |
| PDF7 | 7.28 | 7.68 | 8.56 | 4.62 | 5.15 | 2.76 | | |

Table 7: Viscosity comparison of clear vs. opaque syrup (in centipois per second) at 77% dry solid

| | | | | | |
|---|---|---|---|---|---|
| 1st sample | 7530 | 3722 | 2166 | 1363 | 864 |
| drum 1 | 1744 | 1053 | 686 | 455 | 312 |
| drum 2 | 3478 | 1973 | 1195 | 756 | 503 |
| drum 3 | 1313 | 801 | 506 | 347 | 240 |
| drum 4b | 1856 | 1134 | 713 | 474 | 309 |
| drum 5 | 3366 | 1898 | 1175 | 759 | 509 |
| drum 6 | 1263 | 766 | 499 | 344 | 237 |
| bucket 1 | 3225 | 1856 | 1150 | 857 | 586 |

| Sample | 80°F Viscosity (in cps) | 90°F Viscosity (in cps) | 100°F Viscosity (in cps) | 110°F Viscosity (in cps) | 120°F Viscosity (in cps) |
|---|---|---|---|---|---|
| PDF12 | 3853 | 2133 | 1297 | 827 | 553 |
| PDF7 | 15010 | 10690 | 7740 | 5870 | 4460 |

Table 8: The digestion rates of clear vs. opaque syrup (in % of glucose released after 0-24 hours)

| Sample | 0 hours | 4 hours | 8 hours | 24 hours |
|---|---|---|---|---|
| PDF 12 | 0.0% | 25.6% | 31.0% | 42.5% |
| PDF6 | 3.6% | 25% | 31.3% | 43.6% |

**Example 8, Clear syrups made at high temperatures with enzyme preparations from various *Leuconostoc* strains**

Enzyme preparations were obtained from 3 different *Leuconostoc* strains using methods described in Example 1. A total of 3 kg sugar substrate (sucrose:maltose=9: 1) was dissolved in city water to final concentrations of 50% dry solid w/w or 52% dry solid w/w including enzyme addition. Reactions were carried out at 50 °C or 52 °C (Table 9 below). Aliquots were drawn at the end of each reaction and their sugar profiles and in vitro digestibility were determined as described.

As shown in Table 9, all enzyme preparations completed the reactions. The resulting syrup exhibited similar chemical composition and in vitro digestion rates.

**Table 9. Reaction condition, sugar profile and in vitro digestibility of syrups made at high temperatures**

| Strain | Reaction condition | | | Sugar profile | | | | % Digested after 28 hours |
|---|---|---|---|---|---|---|---|---|
| | Temperature | Concentration | Enzyme dosage | Fructose | Leucrose | DP2-6 | DP7-12 | |
| | C | % w/w | U/g | % | % | % | % | % |
| NRRL-B-30821 | 50 | 52 | 3 | 38.2 | 14.0 | 8.9 | 39.0 | 42.7 |
| NRRL-B-30894 | 52 | 50 | 3.1 | 36.5 | 12.6 | 9.7 | 41.2 | 41.1 |
| NRRL-B-21297* | 52 | 50 | 5 | 36.1 | 13.5 | 9.5 | 40.9 | 45.3 |
| * Leathers et al; Journal of Microbiology and Biotechnology, 1997, pages 278-283 | | | | | | | | |

**Claims**

1. A method of making a substantially clear syrup comprising reacting more than one substrate at a concentration of at least 40 % w/w with at least one alternan sucrase enzyme at a temperature of greater than 45°C to form a substantially clear syrup, wherein the substrates comprise sucrose and an acceptor, and wherein the acceptor is selected from a sugar or sugar alcohol having free hydroxyl groups at one or more carbon positions numbers 2, 3, and 6 that can accept a glucose unit from sucrose.

2. The method according to claim 1, wherein the at least one alternan sucrase and substrates are held at a temperature greater than 45°C for less than 12 hours.

3. The method according to claim 1, wherein the enzyme has a concentration of less than 8 units/gram substrate.

4. The method according to claim 1, wherein the syrup at a concentration of 20% w/w has a transmittance at 650 nm of greater than 93%.

5. The method according to claim 1, wherein the syrup comprises less that 0.5% alternan that is greater than DP12.

6. The method according to claim 1, wherein the syrup has a viscosity of less than 14000 cps at 27°C (80°F) and 77% dry solid concentration.

7. The method according to claim 1 wherein the at least one alternan sucrase enzyme is obtained from lactic acid bacteria.

8. The method according to Claim 1 wherein the at least one alternan sucrase enzyme is obtained from *Leuconostoc mesenteroides.*

9. The method according to Claim 1 wherein the at least one alternan sucrase enzyme is obtained from a strain selected from the group consisting of *Leuconostoc mesenteroides* strains NRRL B 1355, 23185, 23186, 23188, 23311, and 21297.

10. The method according to Claim 1 wherein the at least one alternan sucrase enzyme is produced recombinantly.

11. A syrup made according to the method of claim 1.

12. A food or beverage composition comprising the syrup of claim 11.

**13.** A method of making a food or beverage comprising mixing one or more ingredients with a substantially clear syrup made by reacting more than one substrate at a concentration of at least 40 % w/w with at least one alternan sucrase enzyme at a temperature of greater than 45°C, wherein the substrates comprise sucrose and an acceptor, and wherein the acceptor is selected from a sugar or sugar alcohol having free hydroxyl groups at one or more carbon positions numbers 2, 3, and 6 that can accept a glucose unit from sucrose.

**14.** The method according to Claim 13 wherein the beverage is a sports drink.

**15.** The method according to claim 13 wherein the at least one alternan sucrase enzyme is obtained from lactic acid bacteria.

**16.** The method according to Claim 13 wherein the at least one alternan sucrase enzyme is obtained from *Leuconostoc mesenteroides.*

**17.** The method according to Claim 13 wherein the alternan sucrase enzyme is obtained from a strain selected from the group consisting of *Leuconostoc mesenteroides* strains NRRL B 1355, 23185, 23186, 23188, 23311, and 21297.

**18.** The method according to Claim 13 wherein the at least one alternan sucrase enzyme is produced recombinantly.

**19.** The method according to claim 13, wherein at least one of the ingredients is a vitamin.

**20.** The process according to claim 13, wherein at least one of the ingredients is a high intensity sweetener.

**21.** A food made by the method according to claim 13.

**22.** A beverage made by the method according to claim 13.

**Patentansprüche**

**1.** Verfahren zum Herstellen eines im Wesentlichen klaren Sirups, umfassend das Reagieren von mehr als einem Substrat bei einer Konzentration von mindestens 40% Gew./Gew. mit mindestens einem Alternansucrase-Enzym bei einer Temperatur von höher als 45°C, um einen im Wesentlichen klaren Sirup zu bilden, wobei die Substrate Saccharose und einen Akzeptor umfassen, und wobei der Akzeptor ausgewählt ist aus einem Zucker oder Zuk-keralkohol mit freien Hydroxylgruppen an einer oder mehreren Kohlenstoffpositionsnummer(n) 2, 3, und 6, die eine Glukoseeinheit von Saccharose annehmen können.

**2.** Das Verfahren nach Anspruch 1, wobei die mindestens eine Alternansucrase und die Substrate bei einer Temperatur von höher als 45°C für weniger als 12 Stunden gehalten werden.

**3.** Das Verfahren nach Anspruch 1, wobei das Enzym eine Konzentration von weniger als 8 Einheiten/Gramm Substrat aufweist.

**4.** Das Verfahren nach Anspruch 1, wobei der Sirup bei einer Konzentration von 20% Gew./Gew. eine Transmission bei 650 nm von größer als 93% aufweist.

**5.** Das Verfahren nach Anspruch 1, wobei der Sirup weniger als 0,5% Alternan, welches größer ist als DP12, umfasst.

**6.** Das Verfahren nach Anspruch 1, wobei der Sirup eine Viskosität von weniger als 14.000 cPs bei 27°C (80°F) und 77% Trockengewichtskonzentration aufweist.

**7.** Das Verfahren nach Anspruch 1, wobei das mindestens eine Alternansucrase-Enzym aus Milchsäurebakterien erhalten ist.

**8.** Das Verfahren nach Anspruch 1, wobei das mindestens eine Alternansucrase-Enzym erhalten ist aus *Leuconostoc mesenteroides.*

**9.** Das Verfahren nach Anspruch 1, wobei das mindestens eine Alternansucrase-Enzym aus einem Stamm, ausgewählt aus der Gruppe bestehend aus *Leuconostoc mesenteroides* Stämme NRRL B 1355, 23185, 23186, 23188, 23311, und 21297, erhalten ist.

10. Das Verfahren nach Anspruch 1, wobei das mindestens eine Alternansucrase-Enzym rekombinant hergestellt ist.

11. Sirup, hergestellt nach dem Verfahren von Anspruch 1.

12. Lebensmittel- oder Getränke-Zusammensetzung, umfassend den Sirup gemäß Anspruch 11.

13. Verfahren zum Herstellen eines Lebensmittels oder Getränks, umfassend das Mischen von einem oder mehreren Inhaltsstoffen mit einem im Wesentlichen klaren Sirup, der hergestellt ist durch Reagieren von mehr als einem Substrat bei einer Konzentration von mindestens 40% Gew./Gew. mit mindestens einem Alternansucrase-Enzym bei einer Temperatur von höher als 45°C, wobei die Substrate Saccharose und einen Akzeptor umfassen, und wobei der Akzeptor ausgewählt ist aus einem Zucker oder Zuckeralkohol mit freien Hydroxylgruppen an einer oder mehreren Kohlenstoffpositionsnummer(n) 2, 3, und 6, die eine Glukoseeinheit von Saccharose annehmen können.

14. Das Verfahren nach Anspruch 13, wobei das Getränk ein Sportgetränk ist.

15. Das Verfahren nach Anspruch 13, wobei das mindestens eine Alternansucrase-Enzym aus Milchsäurebakterien erhalten ist.

16. Das Verfahren nach Anspruch 13, wobei das mindestens eine Alternansucrase-Enzym aus *Leuconostoc mesenteroides* erhalten ist.

17. Das Verfahren nach Anspruch 13, wobei das Alternansucrase-Enzym aus einem Stamm, ausgewählt aus der Gruppe bestehend aus *Leuconostoc mesenteroides* Stämme NRRL B 1355, 23185, 23186, 23188, 23311, und 21297, erhalten ist.

18. Das Verfahren nach Anspruch 13, wobei das mindestens eine Alternansucrase-Enzym rekombinant hergestellt ist.

19. Das Verfahren nach Anspruch 13, wobei mindestens einer der Inhaltsstoffe ein Vitamin ist.

20. Das Verfahren nach Anspruch 13, wobei mindestens einer der Inhaltsstoffe ein hochintensiver Süßstoff ist.

21. Lebensmittel, hergestellt durch das Verfahren gemäß Anspruch 13.

22. Getränk, hergestellt durch das Verfahren gemäß Anspruch 13.

**Revendications**

1. Procédé de fabrication d'un sirop sensiblement transparent comprenant la réaction de plus d'un substrat à une concentration d'au moins 40 % p/p avec au moins une enzyme alternane-saccharase à une température de plus de 45 °C pour former un sirop sensiblement transparent, dans lequel les substrats comprennent du saccharose et un accepteur, et dans lequel l'accepteur est choisi parmi un sucre ou un alcool de sucre ayant des groupes hydroxyle libres en une ou plusieurs des positions de carbone numéros 2, 3 et 6 qui peuvent accepter une unité glucose du saccharose.

2. Procédé selon la revendication 1, dans lequel l'au moins une alternanesaccharase et les substrats sont maintenus à une température supérieure à 45 °C pendant moins de 12 heures.

3. Procédé selon la revendication 1, dans lequel l'enzyme a une concentration de moins de 8 unités/gramme de substrat.

4. Procédé selon la revendication 1, dans lequel le sirop à une concentration de 20 % p/p a une transmittance à 650 nm de plus de 93 %.

5. Procédé selon la revendication 1, dans lequel le sirop comprend moins de 0,5 % d'alternane qui est supérieure à DP12.

6. Procédé selon la revendication 1, dans lequel le sirop a une viscosité de moins de 14 000 cps à 27 °C (80 °F) et une concentration en solides secs de 77 %.

7. Procédé selon la revendication 1, dans lequel l'au moins une enzyme alternane-saccharase est obtenue à partir de bactéries lactiques.

8. Procédé selon la revendication 1, dans lequel l'au moins une enzyme alternane-saccharase est obtenue à partir de *Leuconostoc mesenteroides.*

9. Procédé selon la revendication 1, dans lequel l'au moins une enzyme alternane-saccharase est obtenue à partir d'une souche choisie parmi le groupe consistant en les souches de *Leuconostoc mesenteroides* NRRL B 1355, 23185, 23186, 23188, 23311 et 21297.

10. Procédé selon la revendication 1, dans lequel l'au moins une enzyme alternane-saccharase est produite par voie recombinante.

11. Sirop fabriqué conformément au procédé selon la revendication 1.

12. Composition d'aliment ou de boisson comprenant le sirop selon la revendication 11.

13. Procédé de fabrication d'un aliment ou d'une boisson comprenant le mélange d'un ou plusieurs ingrédients avec un sirop sensiblement transparent fabriqué par réaction de plus d'un substrat à une concentration d'au moins 40 % p/p avec au moins une enzyme alternane-saccharase à une température de plus de 45 °C, dans lequel les substrats comprennent du saccharose et un accepteur, et dans lequel l'accepteur est choisi parmi un sucre ou un alcool de sucre ayant des groupes hydroxyle libres en une ou plusieurs des positions de carbone numéros 2, 3 et 6 qui peuvent accepter une unité glucose du saccharose.

14. Procédé selon la revendication 13, dans lequel la boisson est une boisson pour le sport.

15. Procédé selon la revendication 13, dans lequel l'au moins une enzyme alternane-saccharase est obtenue à partir de bactéries lactiques.

16. Procédé selon la revendication 13, dans lequel l'au moins une enzyme alternane-saccharase est obtenue à partir de *Leuconostoc mesenteroides.*

17. Procédé selon la revendication 13, dans lequel l'au moins une enzyme alternane-saccharase est obtenue à partir d'une souche choisie parmi le groupe consistant en les souches de *Leuconostoc mesenteroides* NRRL B 1355, 23185, 23186, 23188, 23311 et 21297.

18. Procédé selon la revendication 13, dans lequel l'au moins une enzyme alternane-saccharase est produite par voie recombinante.

19. Procédé selon la revendication 13, dans lequel au moins un des ingrédients est une vitamine.

20. Procédé selon la revendication 13, dans lequel au moins un des ingrédients est un édulcorant haute intensité.

21. Aliment fabriqué par le procédé selon la revendication 13.

22. Boisson fabriquée par le procédé selon la revendication 13.

Fig. 1

## REFERENCES CITED IN THE DESCRIPTION

**Patent documents cited in the description**

- US 6570065 B
- WO 2004023894 A1
- WO 2004068966 A1
- WO 2005089483 A2

**Non-patent literature cited in the description**

- **GILLES JOUCLA.** Characterization of alternansu-crase from Leuconostoc mesenteroides NNRL B-1355: rational and random approach to design of novel glucansucrase. *Doctroal Dissertation, Ingenier INSA, Toulouse, France,* 2003
- **SANZ et al.** *J. Agric. Food Chem.,* 2005, vol. 53, 5911-5916
- **GILLES JOUCLA.** *Doctroal Dissertation, Ingenier INSA, Toulouse, France,* 2003
- **LEATHERS et al.** *Journal of Microbiology and Biotechnology,* 1997, 278-283